# EUROPEAN PATENT APPLICATION

(11) **EP 1 661 622 A1**
(43) Date of publication of application: **31.05.2006**
(21) Application number: 05290447.1
(22) Date of filing: 28.02.2005
(51) Int. Cl.: B01L 3/00, G01N 15/06, C12M 1/00

(54) **Apparatus and method for capturing minute objects floating on a fluid, for example cells**

(30) Priority: 25.11.2004 JP 2004340685
(71) Applicant: FUJITSU LIMITED, Kawasaki-shi, Kanagawa 211-8588 (JP)
(72) Inventor: Nishiyama, Shusaku, c/o Fujitsu Limited, Kawasaki-shi Kanagawa 211-8588 (JP); Ite, Yasuhiro, c/o Fujitsu Limited, Kawasaki-shi Kanagawa 211-8588 (JP)
(74) Representative: Kreutzer, Ulrich

(57) **Abstract**

A capturing apparatus that captures plural minute objects that float on a fluid (L), includes a capturing part (110) having plural attraction ports (112b) that can attract each object, and an induction part (130) that creates a flow in the fluid used to induce the plural objects to the attraction ports.

## Description

The present invention relates generally to capture of minute floating objects that spread in the fluid. The present invention is suitable, for example, for a capturing apparatus and method that capture minute floating cells in a drug discovery system that investigates reactions of biogenetic cells, such as leukocytes' antibody generations, for use in a medical field. The "drug discovery system", as used herein, generally means a system that processes a cell, e.g., injects extrinsic gene and medication solutions using a fine needle or a capillary into a cell, then cultivates each processed cell, independently evaluate or process the cell (e.g., by screening and antibody extraction).

Recently, opportunities of using cells, into which genes and medication are injected, have increased in the field of regenerative medicine and genome-based drug discovery, etc. Unlike the research application, it is necessary in this medical application to previously determine a combination between a cell and an introduced material and to independently evaluate each cell, e.g., observe whether or not there is an effect expression in a single cell. In addition, the medical application requires a predetermined throughput to be maintained in processing a large amount of cells.

A transgenetic method includes a biological approach, such as a vector method, a chemical approach, such as a transfection, and a physical approach, such as an electroporation, a particle gun and an injection. The biological and chemical approaches are not suitable for the medical application because they limit combinations between cells and introduced materials. On the other hand, the physical approach is known as a method that does not limit the combinations. In particular, the injection approach (see, for example, Japanese Patent Applications, Publication Nos. 8-33477 and 2000-23657) has a high introduction success rate as widely used for artificial inseminations, and is likely to be adopted as a next-generation transgenetic method. According to the prior art injection approach, a skilled operator uses a microscope to introduce a material from a needle tip into a cell while minimizing damages to the cell. Since the injection becomes difficult when the cells are maintained to freely move on a laboratory dish, a method is proposed which attracting and fixing multiple cells at the same time via the porous membrane and the back of the filter (see, for example, Japanese Patent Application, Publication No. 2-117380).

The general cell attraction method proposed by Japanese Patent Application, Publication No. 2-117380 attracts the cells simultaneously and improves the operability if all the cells are located regular intervals apart from corresponding attraction ports. However, if the cells distribute among the attraction ports, the attraction for the predetermined time enables some attraction ports to attract the cells but other attraction cells not to attract the cells. Then, the attraction should continue until all the attraction ports attract the cells and materials are introduced to them. The long attraction possibly drags the cell into the attraction port and damages the cell. In addition, the process efficiency lowers since it is not possible to send the injected cells sequentially to the subsequent process. On the other hand, although the constant attraction time period for the attraction ports for the cells would prevent damages of the cells, the attraction ports that do not attract the cells lower the throughput. The recently proposed cell induction technology called the laser tweezers does not improve the throughput drastically because the laser tweezers induce the cells one by one.

Accordingly, it is an exemplary object of the present invention to provide a capturing apparatus and method, which improve the working efficiency while adopting the injection approach that has no restriction to a combination of a cell and an introduced material, and has a high success rate.

A capturing apparatus according to one aspect of the present invention that captures plural minute objects that float on a fluid includes a capturing part having plural attraction ports that can attract each object, and an induction part that creates a flow in the fluid used to induce the plural objects to the attraction ports. This capturing apparatus creates the flow used to induce the plural objects, and improves the capture efficiency more than the individual induction.

The capturing apparatus may further include a container that houses the fluid and the plural minute objects, wherein the induction part includes a member that inclines the container. Alternatively, the induction section includes means for blowing the air to the cells, and another means. The induction part may create the flow in two orthogonal directions, so as to induce the object two-dimensionally.

The capturing apparatus may further include a detector that detects the attraction ports and the objects, and a controller that determines a direction to be induced and/or an inducing force used for the inducing section to induce the object based on a detection result by the detector. For example, in the embodiment that inclines the container, the capturing apparatus may further include a detector that detects the attraction ports and the objects, and a controller that determines an inclining direction and/or an inclining angle of the cells used for the inducing section to induce the object based on a detection result by the detector. Thereby, the induction of the object may be automated. In particular, the controller adjusts the direction and/or the induction force (such as an inclination angle of the container and blowing force) during an induction of the objects so that the attraction port can attract the object without fail, and the capture efficiency improves. In inducing the plural uncaptured objects, the controller can adjust the induction force in accordance with the distribution shape and size of the plural uncapturing attraction ports for the efficient capturing process, because the objects do not maintain the initial distribution during the induction and the uncapturing attraction ports do not maintain the distribution after attracting the objects.

A capturing method according to another aspect of the present invention that captures plural minute objects that float on a fluid includes the steps of calculating a first center of a zone in which the plural minute objects exist, and a second center of a zone in which plural attraction ports exist which have not yet attracted the objects among plural attraction ports that can attract the objects, and creating a flow in the fluid from the first center to the second center. This capturing method efficiently captures plural objects at the attraction ports by moving the objects from the first center (such as a center of the rectangular zone in which plural uncaptured objects exist and a center of gravity of the plural uncaptured objects) to the second center (such as a center of the rectangular zone in which plural uncapturing attraction ports exist and a center of gravity of the plural uncapturing attraction ports). Here, the "first center" covers the first center and its vicinity (such as within a range five times as large as the object's diameter from the first center), and the "second center" covers the second center and its vicinity (such as within a range five times as large as the attraction port's diameter from the first center). The range of the vicinity is an attractable range of the object by the attraction port, and changes in accordance with the cell's induction speed.

Other objects and further features of the present invention will become readily apparent from the following description of the preferred embodiments with reference to accompanying drawings.
FIG. 1 is a schematic block diagram of a cell processing apparatus according to a first embodiment of the present invention.
FIG. 2 is a schematic plane view of the capturing part in the cell processing apparatus shown in FIG. 1.
FIG. 3 is a schematic plane view showing one exemplary action of the capturing part in the cell processing apparatus shown in FIG. 1.
FIG. 4 is a schematic sectional view of the cell processing apparatus shown in FIG. 3.
FIGs. 5A and 5B are schematic plane and sectional views of a variation of an inclination mechanism in the cell processing apparatus shown in FIG. 1.
FIG. 6 is a schematic plane view for explaining actions of the inclination mechanism shown in FIGs. 5A and 5B.
FIG. 7 is a schematic block diagram of a cell processing apparatus according to a second embodiment of the present invention.
FIG. 8 is a flowchart for explaining an operation of the cell processing apparatus shown in FIG. 7.
FIG. 9 is a schematic block diagram of a cell processing apparatus according to a third embodiment of the present invention.
FIG. 10 is a schematic plane view showing one exemplary action of the capturing part in the cell processing apparatus shown in FIG. 9.
FIG. 11 is a schematic sectional view of the cell processing apparatus shown in FIG. 10.
FIGs. 12A and 12B are schematic plane and sectional views of a variation of an inclination mechanism shown in FIGs. 5A and 5B.
FIG. 13 is a schematic block diagram of a cell processing apparatus according to a fourth embodiment of the present invention.
FIG. 14 is a flowchart for explaining an operation of the cell processing apparatus shown in FIG. 13.
FIG. 15 is a schematic plane view for explaining a calculation method of centers of the cells and attraction ports in FIG. 14.
FIG. 16 is a schematic plane view for explaining another calculation method of centers of the cells and attraction ports in FIG. 14.
FIG. 17 is a schematic perspective view for explaining another method for creating a flow in the fluid.

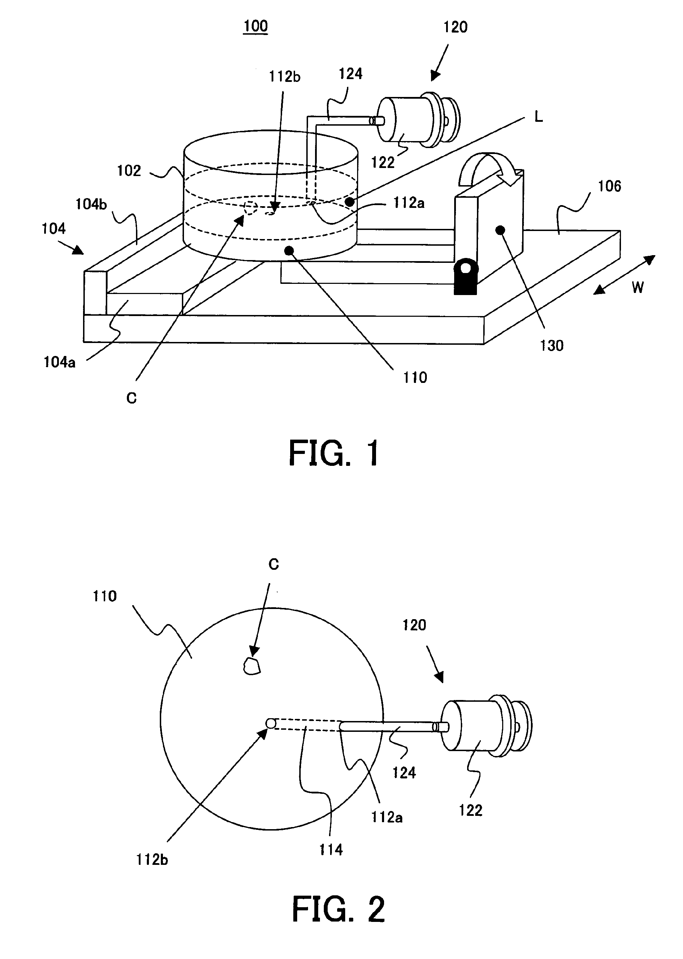

A description will now be given of a cell processing apparatus 100 according to a first embodiment of the present invention, with reference to the accompanying drawings. The cell processing apparatus 100 provides a predetermined process (for example, an injection process of an object using a capillary) to capture minute objects applicable to the present invention. The minute objects spread and can float on a fluid L, such as the cell suspension and medium. Here, FIG. 1 is a schematic perspective view of the cell processing apparatus 100. The cell processing apparatus 100 includes, as shown in FIG. 1, a container 102, such as a laboratory dish, a support part 104, a base 106, a capturing base 110, an attraction part 120, and an inclination mechanism 130.

The container 102 is a cylindrical liquid bath that houses the fluid L and the plural cells C that float on the fluid L, and is made, for example, of plastic. The container 102 may be a channel. The container 102 is connected to one or more supply parts (not shown) that supply the cells C and the fluid L. The fluid L is such an amount that the cells C float on the fluid L without contacting the capturing base 110 during the normal time period and the cells C are restricted on the capturing base 110 during the attraction time period. Since the fluid L flows in the capturing base 110 from the attraction ports 112b, which will be described later, the sufficient amount of the fluid L needs to be supplied to the container 102 so that the cells C can float without contacting the capturing base 110 even after the fluid L flows in the capturing base 110. For example, the container 102 is provided with a sensor that detects the height of the fluid L, and the container 102 may be configured to receive the fluid L from the supply part (not shown) when the sensor detects that the fluid amount is lower than the predetermined height. The container 102 is connected to a processing part (not shown) that injects, using a capillary, a predetermined material into the plural cells captured by the capturing part, which will be described later. Moreover, the container 102 may be connected to a channel that feeds the cells into which the predetermined material, such as a DNA, has already been injected (or processed cells) to the later recovery part.

The support part 104 includes a bottom support 104a that supports part of the bottom of the container 102, and a side support 104b that supports part of the side of the container 102. The bottom support 104a and the side support 104b have a plate shape, and a sectionally L-shape as in FIG. 1 in this embodiment. The support part 104 supports the container 102 so that the container 102 can incline or rotate. The support part 104 may be integrated with the base 106. The support part 104 preferably includes a restriction member that restricts a movement of the container 102 in a direction W, thereby preventing the container 102 from rolling in the direction W during an inclination and from being disengaged from the support part 104.

The base 106 fixes the support part 104, and supports the inclination mechanism 130 rotatably.

The capturing base 110 is a cylindrical member that fits the inner surface of the container 102, and serves as the capturing part together with the attraction part 120. The capturing base 110 is made of a material, such as plastic, which has a specific gravity greater than the fluid L and the cell C and does not contaminate the fluid L. Since the capturing base 110 fits the inner surface of the container 102, the capturing base 110 does not move in the container 102. This configuration does not move a pipe 124, and stabilizes the attractions. In addition, this configuration does not cause the fluid L to leak between the container 102 and the capturing base 110. The capturing base 110 may be part of the container 102. The capturing base 110 includes plural connection ports 112a, plural attraction ports 112b, and plural tunnels 114, although FIG. 1 shows one each.

The connection port 112a is a hole connected to the pipe 124 of the attraction part 120. The inside of the connection port 112a is screwed and engageable with the outer ridge of the pipe 124. Thereby, the pipe 124 is prevented from being separated from the capturing base 110 during operation.

Each attraction port 112b is a hole with a diameter of several micrometers, and used to attract and capture the cell C. The number of attraction ports 112b is set to the maximum number of the cells to be captured simultaneously. The attraction ports 112b are arranged in such a lattice shape that the automatic machine can easily handle the cells in the subsequent step, such as a DNA injection, with a predetermined throughput.

The tunnel 114 is a U-shape hole, for example, which connects, as shown in FIG. 2, the connection port 112a and the attraction port 112b. Here, FIG. 2 is a schematic plane view showing a relationship between the capturing base 110 and the attraction part 120. A shape of the tunnel 114 is illustrative, and has a linear shape, for example, when the connection port 112a is formed at the bottom surface of the capturing base 110.

The attraction part 120 can attract and exhaust the cell C, exhaust the fluid L, and includes plural pumps 122 and corresponding pipes 124 in this embodiment, although FIG. 1 shows only one pump 122 and pipe 124 for convenience. The attraction part 120 may include plural pumps 122 corresponding to the number of attraction ports 112 as in this embodiment but at least one pump 122 is enough if each attraction port 112b is provided with a control valve. While the capturing part includes the capturing base 110 and the attraction part 120 in this embodiment, it is optional to provide the capturing base 110. For example, the pipe 124 of the attraction part 120 is made U-shaped in accordance with the shape of the tunnel 114. The exhaust function of the attraction port 112b enables at least one of the attraction ports 112b to serve as an induction part that induces the cell C to a target attraction port 112b. In addition, when plural cells C agglutinate, an adjacent attraction port 112b may pressurize and disperse them.

The pump 122 may use any type as long as at least it provides the attraction (and exhaustion preferably). For example, the pump 122 is an attraction/exhaustion apparatus that includes a cylinder and a plunger that can slide along the axial direction of the cylinder. This embodiment allows the attraction and exhaustion forces of the pump 122 to be adjustable. The attraction force used to capture the cell C without damaging it, and the exhaustion force that releases the cell C are previously set by simulation or experimental results. The attraction force used to draw the cell near the attraction port 112b, and the exhaustion force of the fluid L used to disperse the cells C are made adjustable so as to improve the capturing efficiency.

The pipe 124 is connected to the connection port 112a of the capturing base 110, and includes an elastic tube. In this embodiment, each pump 122 can operate independently. Although the pipe 124 bends in a right angle, this shape is illustrative. If the attraction part 120 is provided below the capturing base 110, the connection port 112a should be formed in the bottom surface of the capturing base 110. However, that case needs means for preventing inflow of the fluid L into the pump 122.

According to this attraction part 120, the plural attraction ports independently attract the cells (for example, by manual operations), and thus the cells C are prevented from being attracted for a long time and from getting damaged. While each pump 122 can operate independently, the attraction action may perform the simultaneous capturing of the cells C. FIG. 2 shows the captured and restrained cell. In this state, an operator stabs a capillary into the cell C and injects a predetermined material, such as DNA. The operability improves since the cell C is fixed.

The capturing part individually releases the injected cells C captured by the plural attraction ports 112b, and can improve the injection efficiency and throughput. For example, the individual stop of the attraction or exhaustion start only at the central attraction port 112b that captures the processed cell C enables the center cell C to be freely movable, to move down by other cell moving means, such as an inclination of the container 102, and separate from other restrained cells C. If the processed and unprocessed cells C are discernable, the entire process flow can be automated. In addition, it is possible to capture and restrain the new cell C individually in the target attraction port 112b.

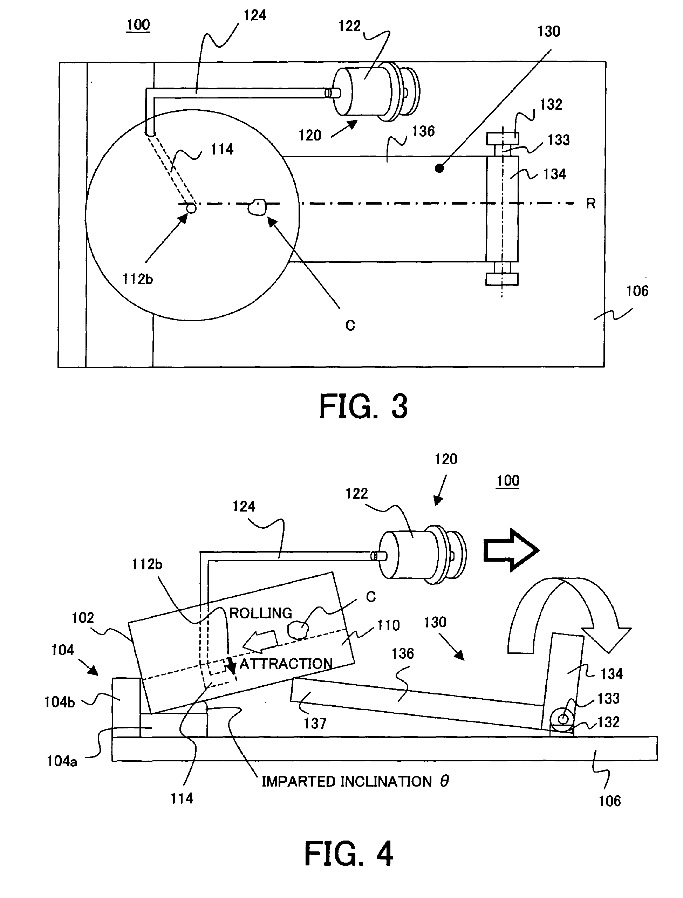

The inclination mechanism 130 serves to incline the container 102 at a predetermined angle in a predetermined direction. The predetermined direction is a direction to induce the cell C, and the predetermined angle corresponds to the induction force used to induce the cell C by gravity. The inclination mechanism 130 has, as shown in FIGs. 1, 3 and 4, a lever structure in this embodiment, and more specifically a pair of bases 132, a power point part 134, and an inclination part 136. Here, FIG. 3 is a schematic plane view showing an inclination of the container 102. FIG. 4 is a schematic sectional view showing an inclination of the container 102. In FIG. 3, the predetermined direction is the direction R, and accords with the line that connects the cell C and the attraction port 112b.

The pair of bases 132, which have a shape that combines a rectangular parallelepiped and a semi-cylinder, are located on the base 106. A shaft 133 is fixed between the pair of bases 132, and the power point part 134 is non-rotatably provided around the shaft 133. The power point part 134 serves as a power point during the inclination of the container and has, but is not limited to, a plate shape in this embodiment. The initial inclination angle of the inclination part 136 from the base 106 is set to 0, and the inclination part 136 contacts the base 106 in FIG. 4. When the inclination part 136 attempts to incline the container 102, the inclination part 136 contacts the container 102 at a contact portion 137 shown in FIG. 4.

For example, when the cell C is located at a position shown in FIG. 2, the cell C cannot be captured quickly even if the pump 122 starts the attraction at the attraction port 112b due to a relatively long distance from the attraction port 112b to the cell C. Accordingly, as shown in FIG. 4, the inclination mechanism 130 inclines the container 102 along the line R shown in FIG. 3. As a result, the cell C rolls to the attraction port 112b by gravity. The inclined angle θ is several degrees in this embodiment, and made variable.

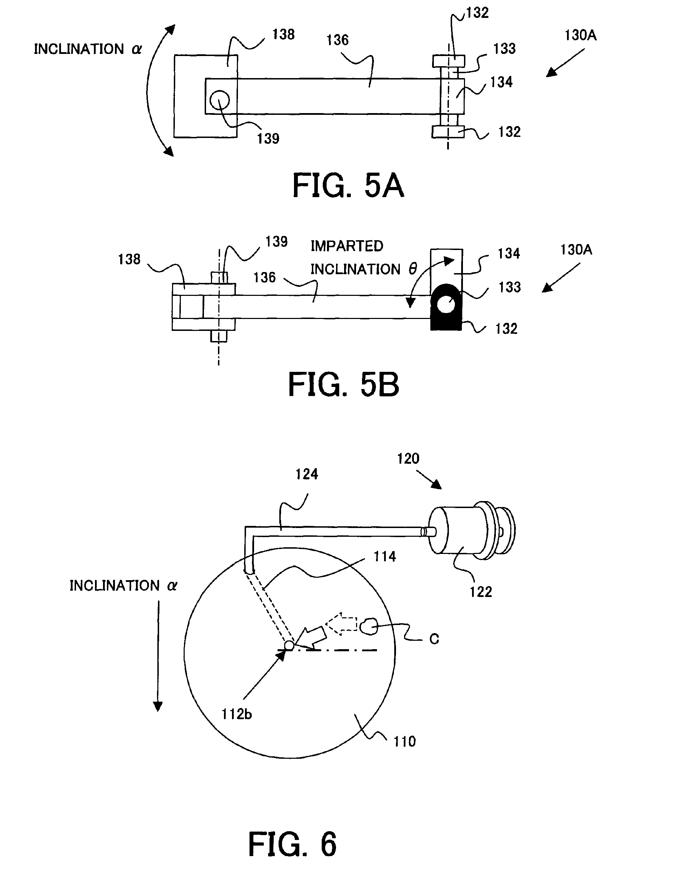

The inclination mechanism 130 may includes a mechanism that rotates the container 102 automatically (such as a rotational rack that supports the container 102 and is rotatable relative to the base 106) or the container 102 may be rotated manually. As a result, it is easy to align the cell and the attraction port 112b with the inclination direction R. The former example will be described with reference to FIGs. 5A, 5B and 6. Here, FIG. 5A is a schematic plane view of the inclination mechanism 130A, and FIG. 5B is a schematic sectional view of the inclination mechanism 130A. FIG. 6 is a schematic plane view showing a movement of the cell on the capturing base 110 when the inclination mechanism 130A is used.

The inclination mechanism 130A is different from the inclination mechanism 130 in that the inclination mechanism 130A further includes a sectionally U-shaped support part 138, and a shaft 139 that attaches the support part 138 rotatable relative to the inclination part 136. As a result, the inclination mechanism 130A can incline the container 102 at the inclination angle θ, and can change the inclination direction or angle α. When the inclination mechanism 130 inclines the container 102, the cell C starts rolling by gravity. Even if the cell C starts rolling in a broken line direction off the attraction port 112b as shown in FIG. 6, the rolling direction can be adjusted to the solid line direction by rotationally adjusting the support part 138 around the shaft 139.

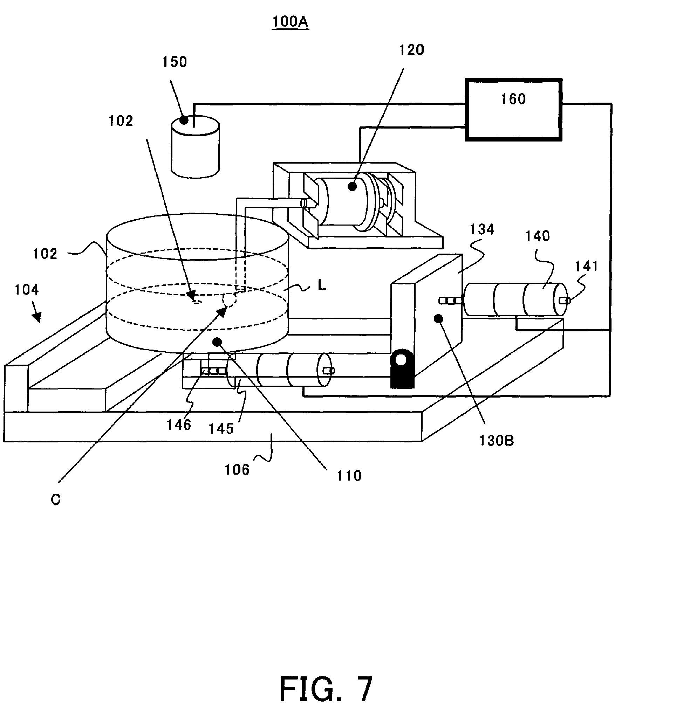

Referring now to FIG. 7, a description will be given of the cell processing apparatus 100A that automates the mechanisms shown in FIGs. 5 and 6. Here, FIG. 7 is a schematic perspective view of the cell processing apparatus 100A. Those elements in FIG. 7, which are corresponding elements in FIG. 1, are designated by the same reference numerals, and a description thereof will be omitted. The cell processing apparatus 100A is different from the cell processing apparatus 100 in that the cell processing apparatus 100A includes an inclination mechanism 130B, a detector 150, and a controller 160.

The inclination mechanism 130B includes a drive part 140 that is connected to the power point part 134 via a connector 141 that can move back and forth, and rotates the power point part 134 around the shaft 133, and a drive part 145 that is connected to the support part 138 via a connector 146 that can move back and forth, and rotates the support part 138 around the shaft 139. The drive parts 140 and 145 include, for example, an air cylinder.

The detector 150 has a field to photograph the capturing base 110, and photographs states of the cells C and the attraction ports 112b. The detector 150 is made, for example, of a CCD camera. While the detector 150 continuously detects them in this embodiment, it can detect them at predetermined time intervals.

The controller 160 has an image processing function that detects, based on the detection results by the detector 150, positions of cells C (such as, shapes, sizes, positions of cells C, whether the cells C move or not, and moving directions) and states (such as, whether the cells are captured by the attraction ports 112b). The controller 160 calculates the inclination direction and the inclination angle by the inclination part 130B based on the detection results. While the controller 160 in this embodiment has an image processing function and serves as an operational part, the image processing unit (or an operational part) may be provided independently of the controller 160, and the controller 160 may utilize a personal computer ("PC") so as to establish the data communications between the image processing unit and the PC. In addition, the drive parts 140 and 145 may have controllers independent of the controller 160.

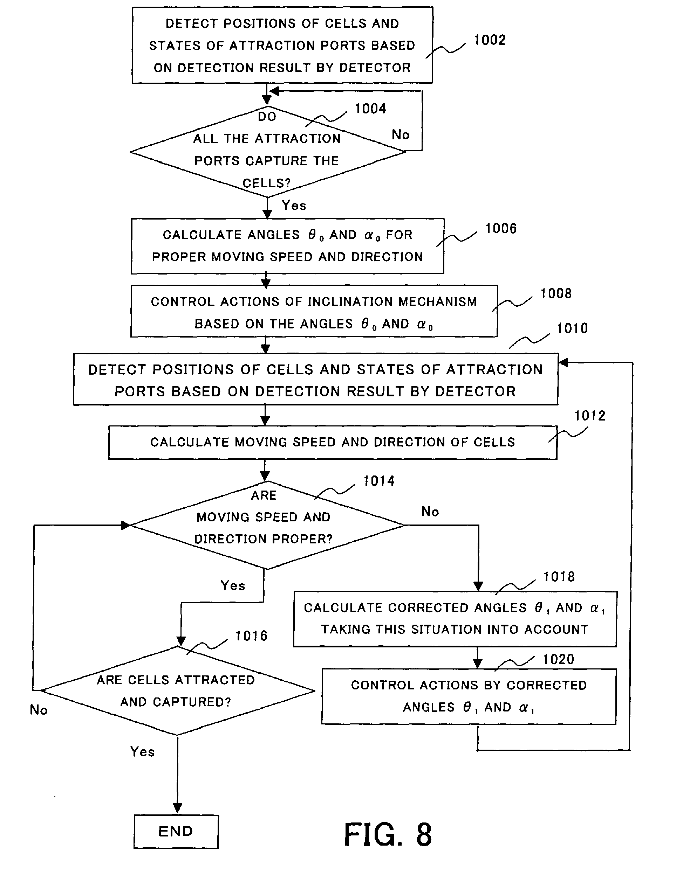

Referring now to FIG. 8, a description will be given of the operation of the cell processing apparatus 100A. Here, FIG. 8 is a flowchart showing an operation of the cell processing apparatus 100A. First, the controller 160 detects the positions of the cells C and the states of the attraction ports 112b based on the detection results by the detector 150 (step 1002), and determines whether all the attraction ports 112b capture the cells C (step 1004). When determining that not all the attraction ports 112b capture the cells C, the controller 160 then calculates angles θ₀ and α₀ used to provide the cells C with proper moving speed and direction (step 1006). The angle θ represents an angle of the power point part 134 relative to the base 106, and the angle α represents an angle of the support part 138 relative to the inclination part 136.

Next, the controller 160 controls the drive parts 140 and 145 of the inclination mechanism 130B based on the calculated angles θ₀ and α₀ (step 1008). As a result, the inclination mechanism 130B inclines the container 102 and the cell C starts rolling, for example, as shown by the broken line in FIG. 6.

Again, the controller 160 determines the positions of the cells C and the states of the attraction ports 112b based on the detection result by the detector 150 (step 1010), and calculates the actual moving speeds and directions of the cells C (step 1012). Then, the controller 160 determines whether the moving speeds and directions are proper (step 1014). The determination of the properness is based on whether the moving direction R of the cell C shown in FIG. 3 accords with the line that connects the center of the cell C to the center of the attraction port 112b, but a slight offset is permissible. A range of the offset depends upon the attractable range of the cell C by the attraction port 112b, and changes in accordance with the cell C's size and induced speed. The cell C's size and moving speed in this embodiment allow offsets up to the five times as large as the diameter of the cell C and five times as large as the diameter of the attraction port 112b.

When determining that the cell C's moving speed and direction are proper (step 1014), the controller 160 maintains the controls in the step 1008 until the attraction port 112b captures the cell C (step 1016). When the attraction port 112b captures the cell C, the capture control process ends and is followed by the injection process using the capillary.

On the other hand, the controller 160 when determining that the cell C's moving speed and direction are proper (step 1014), corrects them to the corrected angles θ₁ and α₁ (step 1018). Next, the controller 160 controls (the drive parts 140 and 145 of) the inclination mechanism 130 based on the corrected angles θ₁ and α₁ (step 1020). As a result, the inclination mechanism 130 inclines the container 102, and the cells start rolling as shown by a solid line shown in FIG. 6. Then, the process returns to the step 1010. This automatic induction process improves the capture efficiency. This embodiment also automates the attraction using the pump 122 for the attraction port 120.

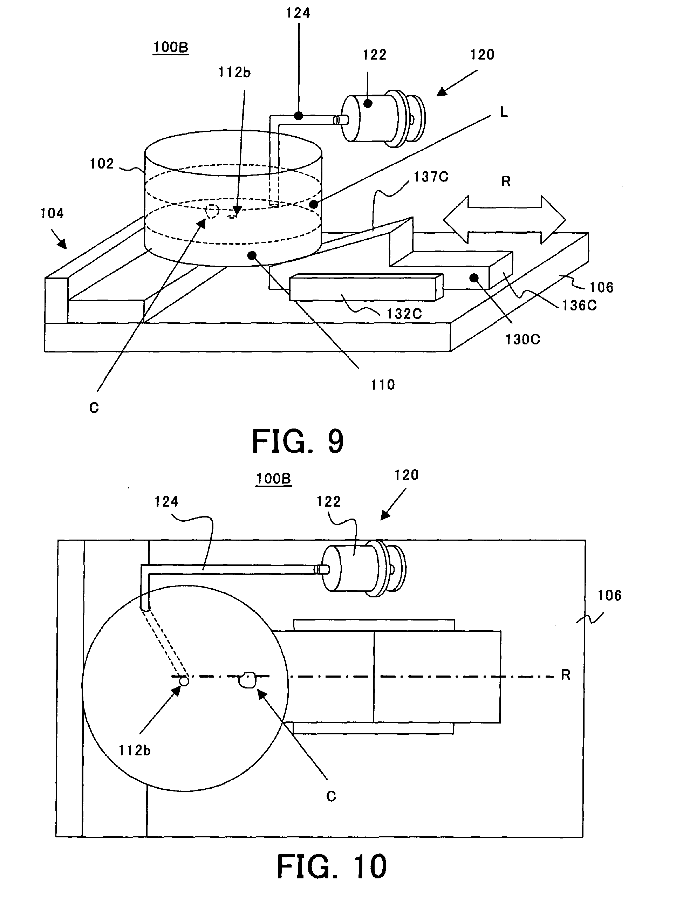

Referring now to FIGs. 9 to 11, a description will be given of a cell processing apparatus 110B according to a third embodiment of the present invention. Here, FIG. 9 is a schematic perspective view of the cell processing apparatus 100A. Those elements in FIG. 9, which are the same as corresponding elements in FIG. 1, are designated by the same reference numerals, and a description thereof will be omitted. The cell processing apparatus 100B is different from the cell processing apparatus 100 in that the cell processing apparatus 100B includes the inclination mechanism 130B different from the inclination mechanism 130. The inclination mechanism 130C does not use the lever, and includes a power point part 132C having a rail function, and a an inclination part 136C that has an approximately triangle section and is movable along the direction R shown in FIG. 9 on the power point part 132C. The inclination part 136C contacts the container 102 on the inclination surface 137C.

The inclination mechanism 130C serves to incline the container 102 at a predetermined angle in the direction R. The direction R is a direction used to induce the cell C, and the movement of the inclination part 136C in the direction R corresponds to the inclination angle. At the initial state, the container 102 is maintained to be parallel (horizontal) to the base 106.

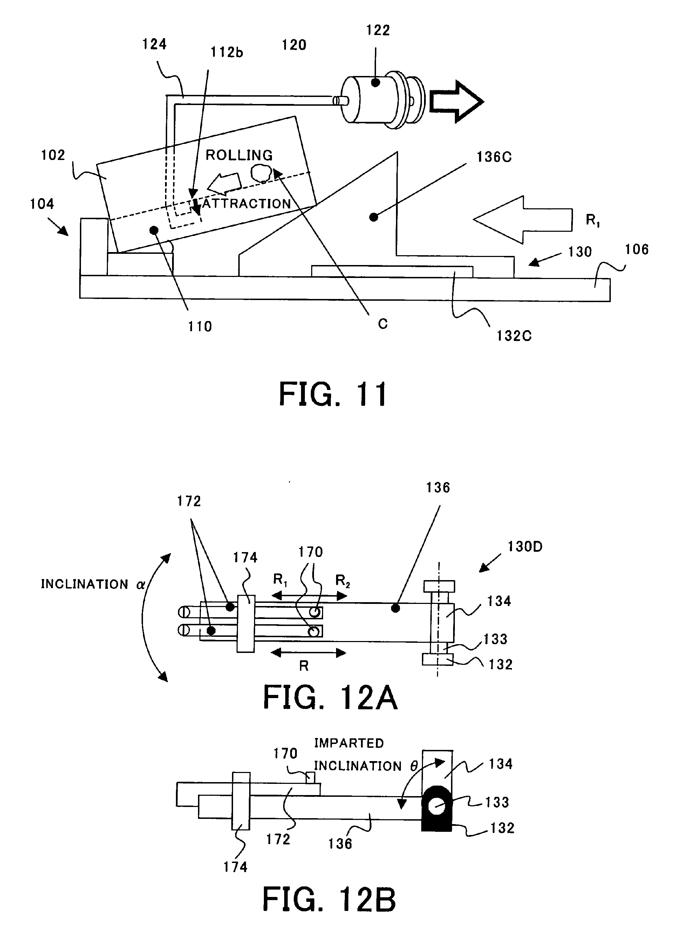

FIGs. 10 and 11 show that the inclination mechanism 130C inclines the container 102. Here, FIG. 10 is a schematic plane view showing that the inclination mechanism 130C moves in a direction R₁ and inclines the container 102. FIG. 11 is a schematic sectional view of that state. As shown in FIG. 10, the cell C and the attraction port 112B are previously set so that they accord with the direction R. The container 102 inclines as shown in FIGs. 3 and 4.

FIGs. 12A and 12B show an inclination mechanism 130D as a variation of FIG. 5. Those elements which are corresponding elements in FIG. 5 are designated by the same reference numerals. Here, FIG. 12A is a schematic plane view of the inclination mechanism 130D, and FIG. 12D is a schematic side view of the inclination mechanism 130D. The inclination mechanism 130D includes a pair of levers 170, a pair of guides 172, and one weight 174. The guides 172 are drivable in the direction R by the levers 170, and rotate the container 102 in the direction α. The guides 172 are configured to move only in the direction R by rails (not shown). During the rotation, a pair of guides 172 move alternately. In an attempt to rotate the container 102 (not shown) in FIG. 12A, for example, the upper lever 170 and guide 172 are driven in a direction R₂ and the lower lever 170 and guide 172 are driven in a direction R₁. The weight 174 restrains the guide 172 and restricts the movement of the lever 170 in the direction R₁. This inclination mechanism 130D operate similar to the inclination mechanism 130.

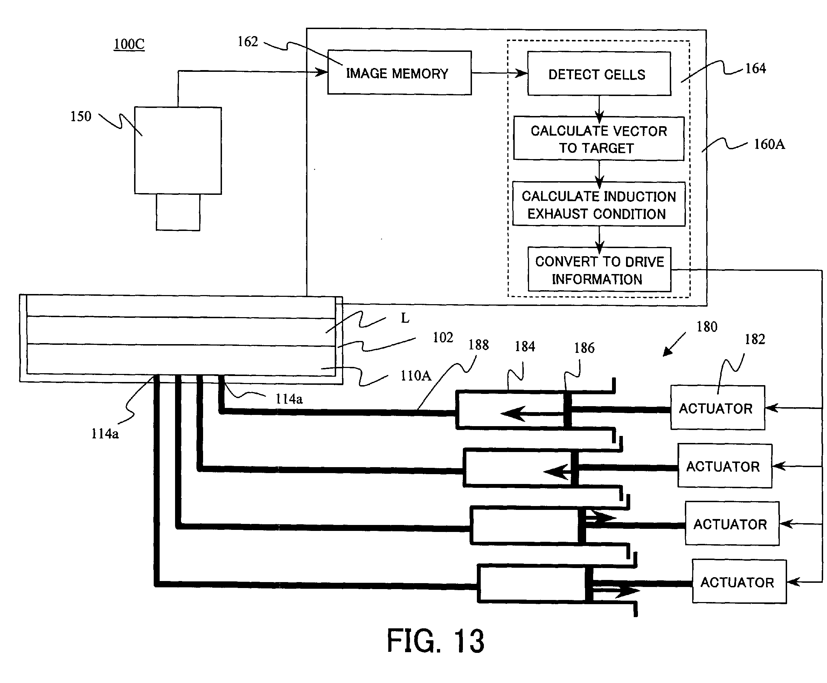

Referring now to FIG. 13 to 18, a description will be given of a cell processing apparatus 100C according to a fourth embodiment of the present invention. Here, FIG. 13 is a schematic block diagram of the cell processing apparatus 100C. The cell processing apparatus 100C is different from the cell processing apparatus 100A in that the cell processing apparatus 100C includes a capturing base 110A, a controller 160A, and an induction part 180.

Similar to the cell processing apparatus 100A, the capturing base 110A is connected to the attraction part 120 and is provided with the attraction ports 112b. However, the capturing base 110A further includes connection ports 114a and induction ports 114b, and an induction part 180 is connected to the connection ports 114a.

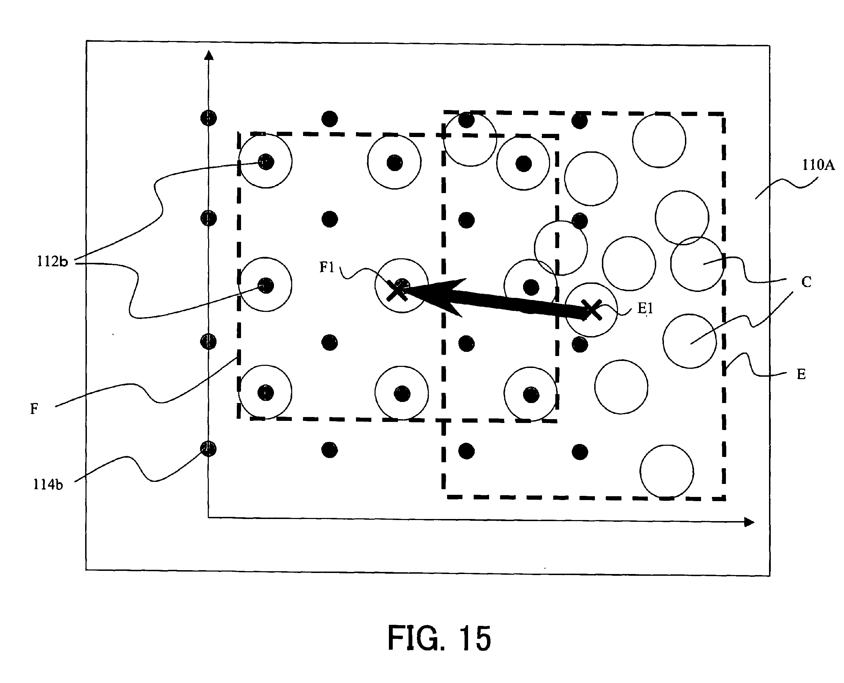

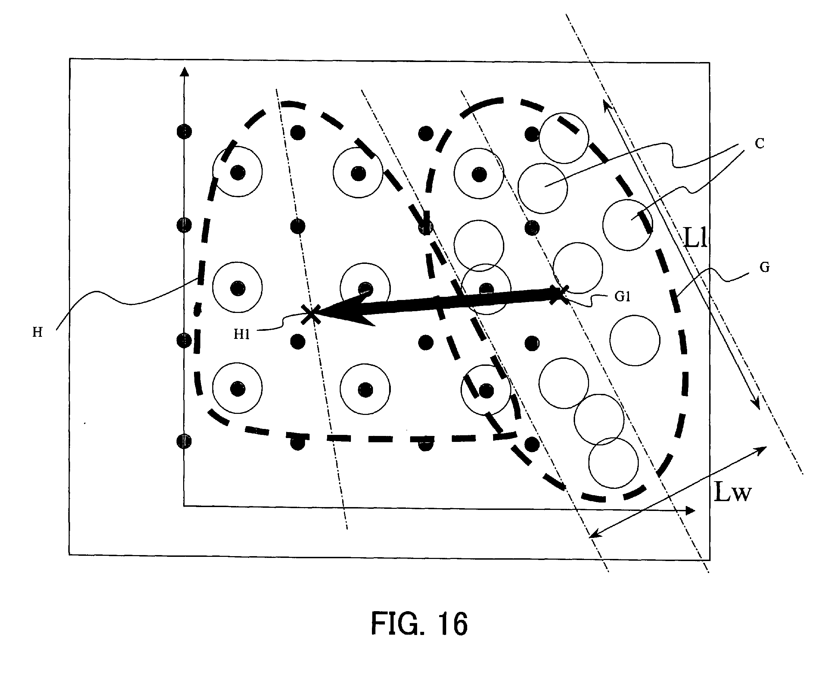

In the capturing base 110A, as shown in FIGs. 15 and 16, which will be described later, plural attraction ports 112b and induction ports 114b are arranged in a matrix shape, plural attraction ports 112b and plural induction ports 114b are offset in the diagonal direction. The attraction port 112b is used to attract the cell C and, if necessary, exhaust the processed cell C or the unprocessed cell C so as to introduce it to the desired attraction port 112b. On the other hand, the induction port 114b attracts and exhausts the fluid L so as to introduce the cell C to the desired attraction port 112b.

In this embodiment, the induction ports 114b open approximately perpendicular to the top surface of the capturing base 110A similar to the attraction ports 112b. While the attraction ports 112b open approximately perpendicular to the top surface of the capturing base 110A so as to facilitate fixtures of the cells C, it is sufficient that the induction ports 114b induce the cells C. Therefore, an alternate embodiment arranges the induction port 114b oblique to the top surface of the capturing base 110A, or opens the induction port 114b approximately perpendicular to the top surface of the capturing base 110A with a wall that shields a certain range of the induction port 114b so that the induction port 114b can apply strong attraction and exhaustion forces in a predetermined direction. The induction port 114b that can apply strong attraction and exhaustion forces in a predetermined direction may be provided rotatably around the capturing base 110A, and the controller 160A may adjust the predetermined direction. A restriction of the direction of the force applied by the induction port 114b can prevent the negative influence on the inductions of other cells C as a result of that the force expands in an isotropic manner.

Similar to the cell processing apparatus 100A, the controller 160A is connected to the detector 150 and plural pumps 122, and controls driving of the attraction part 120 based on the detection results of the detector 150. However, different from the cell processing apparatus 100A, the controller 160A is further connected to the induction part 180, and controls driving of the induction part 180 based on the detection results of the detector 150. FIG. 13 omits the attraction part 120, and shows only a relationship among the detector 150, the controller 160A, and the induction part 180.

As shown in FIG. 13, the controller 160A is implemented by a PC etc., and includes an image memory 162 that stores image information sent from the detector 150, and an image processor 164 that executes control processes over the attraction part 120 and the induction part 180 based on the image information. The image processor 164 detects the cell, calculates a target vector, an induction exhaustion condition, and converts data into drive information, and controls the induction part 180 based on this drive information.

The induction part 180 has a structure similar to that of the attraction part, and includes four pairs of actuators 182, cylinders 184, plungers 186 and pipes 188 in this embodiment, although the number of pairs is illustrative. The actuator 182 drives in the left and right direction in FIG. 13. The plunger 186 is connected to the shaft of the cylinder 184, moves in the axial direction of the cylinder 184, and exhausts and pressurizes the fluid L. When the actuator 182 drives the plunger 186 in the left direction in FIG. 13, the positive pressure applies to (the cells C in) the fluid L through the pipe 188 whereas when the actuator 182 drives the plunger 186 in the right direction in FIG. 13, the negative pressure applies to (the cells C in) the fluid L through the pipe 188.

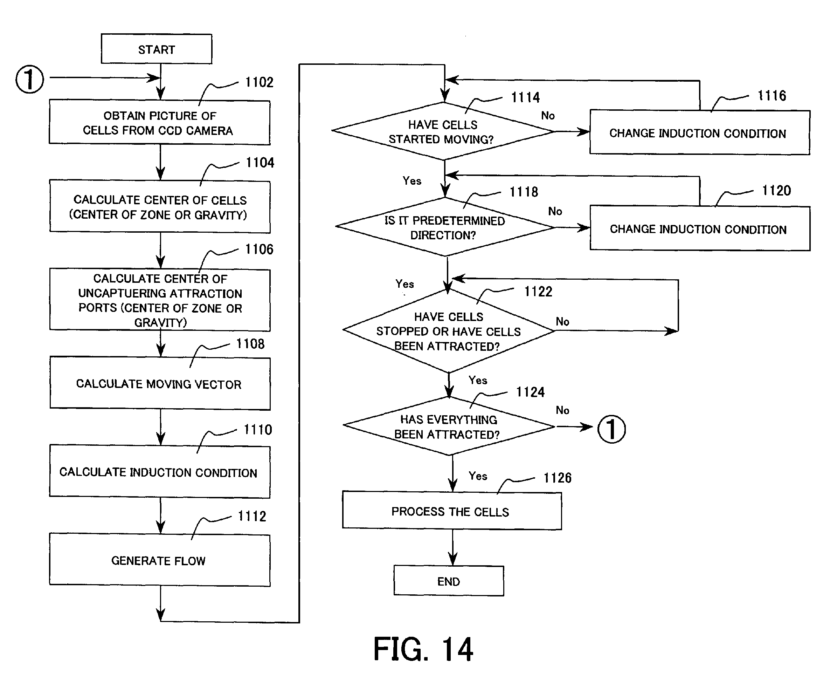

Referring now to FIGs. 14 to 16, a description will be given of an operation of the cell processing apparatus 100C. Here, FIG. 14 is a flowchart for explaining the operation of the cell processing apparatus 100C. First, the image processor 163 in the controller 160A obtains the image of the cells from the detector 150 via the image memory 162 (step 1102).

Next, the image processor 164 calculates the center of the unattracted cells (which is a center of zone or gravity) and the center of the attraction ports that do not attract the cells (which is a center of zone or gravity) (steps 1104 and 1106). The center of the cells is a reference point used to move the entire cells, and the center of the attraction ports is a reference point where the entire cells should reach. As shown in FIG. 15, the center of the zone of the cells is a center E1 of a rectangular zone E in which the cells distribute, and the center of the zone of the attraction ports is a center F1 of a rectangular zone F in which the attraction ports distribute. Here, FIG. 15 is a schematic plane view for explaining the setting of the centers of zones of the unattracted cells and attraction ports 112b that do not attract the cells. A zone shape is not necessarily limited to the rectangle, and may use an arbitrary shape, such as a rhombus. Similarly, as shown in FIG. 16, the center of gravity of the cells is a center G1 of the area G in which the cells exist, and the center of gravity of the attraction ports is a center H1 of the area H in which the attraction ports exist. These centers of gravity are points around which a pair of orthogonal central principal axes can be defined. For example, the center G1 is calculated from the distribution of the cells that exist in the properly set area G having a proper shape (defined by a length L1 and a width LW in this embodiment). Here, FIG. 16 is a schematic plane view for explaining the setting of the centers of gravities of the areas of the unattracted cells and attraction ports 112b that do not attract the cells.

Next, the controller 160 sets a vector used to move the cells as shown in FIGS. 15 and 16 from the two centers calculated in the steps 1104 and 1106 (step 1108). Then, the controller 160 calculates the induction condition by the induction part 180 based on the vector, such as the attraction and exhaustion directions and the attraction and exhaustion forces of the induction part 114b by the induction part 180 (step 1110). The induction condition is set so that the flow in the fluid created by the induction part 180 accords with the vector used to move the cells. The flow speed in the fluid is set to the speed usable for the attraction port for attraction. The flow speed and the induction condition are previously calculated by simulation and stored in the memory (not shown). The controller 160 may obtain the induction condition by referring to the memory after obtaining the centers of the cells and the attraction ports. Next, the controller 160 generates the flow in the fluid based on the induction condition by controlling the operation of the induction part 180 (step 1112).

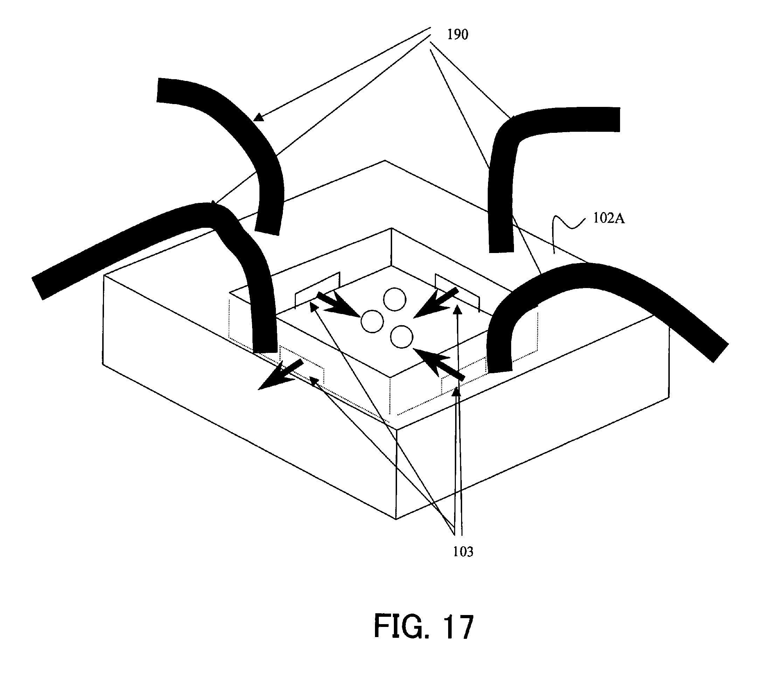

While this embodiment uses the induction part 180, the container 102A may have, as shown in FIG. 17, a tube 190 that exhausts and attracts the fluid L in the two orthogonal directions, and fluid introduction/exhaustion port 103. This embodiment controls the exhaustions and attractions of the fluid L from the tube 190, and creates the flow shown in FIG. 17 in the container 102A.

Next, the controller 160 determines whether the center of the cells has moved and whether the cells start moving based on the detection result by the detector 150 (step 1114), and changes the induction condition if the cells have not moved (step 1116). The controller 160 determines, after determining that the cells have moved (step 1114), whether the movement is a predetermined direction approximately following the vector (step 1118), and changes the induction condition if determining that the movement does not approximately follow the vector (step 1120). In this case, the controller 160 determines whether an offset between the predetermined direction and the vector is within a permissible range, which is freely determined by a user. In inducing the cells, the controller 160 can adjust the induction force in accordance with a distribution shape and a size of the plural uncapturing attraction ports. Since the cells do not maintain the first distribution during the induction and the uncapturing attraction ports also do not maintain the distribution by the attractions of the cells, the continuous control over the induction direction and induction force provides the efficient capture.

When determining that the movement of the cells follows the predetermined direction (step 1118), the controller 160 determines whether the cells have stopped (step 1122). Next, the controller 160 determines whether all the attraction ports capture and attract the cells (step 1124). When determining that not all the attraction ports capture and attract the cells (step 1124), the procedure returns to the step 1102. On the other hand, when determining that all the attraction ports capture and attract the cells (step 1124), the controller 160 performs predetermined process for the cells C, such as the DNA injection (step 1126).

For example, an injector (not shown) stabs a capillary into a cell membrane of the cell C and injects a gene solution and drug solution. The controller of the injector controls the timing, injection amount, injection angle, and injection depth used for the capillary to inject the gene solution and the drug solution into the cell C. The capillary has a sharp tip enough for injection into the cell C, and its diameter is, for example, about 1 µm. Such an injection method can improve the success rate of the injection without incurring a restriction of a combination between the cell and the introduced material.

As discussed above, the above embodiments can induce the cells to plural attraction ports, and improve the capture efficiency. The cell processing apparatus adopts the injection method, and maintains the success rate of the injection without incurring a restriction of a combination between the cell and the introduced material.

Of course, the above embodiment is applicable other embodiments such as that shown in FIG. 1.

Further, the present invention is not limited to these preferred embodiments, and various variations and modifications may be made without departing from the scope of the present invention. For example, a means for creating the flow in the fluid L and for inducing the cell C may be air sending means, such as a blower. The air blasting means if configured to adjust the blasting direction and force can serve as the above inclination mechanism 130.

Thus, the present invention can provide a capturing apparatus and method, which improve the working efficiency while adopting the injection approach that has no restriction to a combination of a cell and an introduced material, and has a high success rate.

## Claims

1. A capturing apparatus that captures plural minute objects that float on a fluid, said capturing apparatus comprising:
a capturing part having plural attraction ports that can attract each object; and
an induction part that creates a flow in the fluid used to induce the plural objects to the attraction ports.

2. A capturing apparatus according to claim 1, further comprising a container that houses the fluid and the plural minute objects,
wherein the induction part includes a member that inclines the container.

3. A capturing apparatus according to claim 2, wherein said induction part includes a member that rotates the container.

4. A capturing apparatus according to claim 1, wherein said induction section includes a member that blows the air to the fluid.

5. A capturing apparatus according to claim 1, wherein said induction part has an induction port that can attracts and exhausts the fluid, and induces the object to a predetermined one of the induction ports.

6. A capturing apparatus according to claim 1, wherein said induction part creates a flow in two orthogonal directions.

7. A capturing apparatus according to claim 1, further comprising:
a detector that detects the attraction ports and the objects; and
a controller that determines a direction to be induced and/or an inducing force used for said inducing section to induce the object based on a detection result by said detector.

8. A capturing apparatus according to claim 7, wherein said controller adjust the direction and/or the induction force during an induction of the objects.

9. A capturing apparatus according to claim 2, further comprising:
a detector that detects the attraction ports and the objects; and
a controller that determines an inclining direction and/or an inclining angle to induce the objects based on a detection result by said detector.

10. A capturing apparatus according to claim 2, further comprising:
a detector that detects the attraction ports and the objects; and
a controller that determines attracting and exhausting directions and attracting and exhausting forces by the attraction ports based on a detection result by said detector.

11. A capturing method that captures plural minute objects that float on a fluid, said capturing method comprising the steps of:
calculating a first center of a zone in which the plural minute objects exist, and a second center of a zone in which plural attraction ports exist which have not yet attracted the objects among plural attraction ports that can attract the objects; and
creating a flow in the fluid from the first center to the second center.

12. A capturing method according to claim 11, wherein the first center is a center of a rectangular zone in which the plural minute objects exist which have not yet been attracted by the attraction ports.

13. A capturing method according to claim 11, wherein the first center is a center of gravity of the plural minute objects that are not attracted by the attraction ports.

14. A capturing method according to claim 11, wherein the second center is a center of a rectangular zone in which the plural attraction ports exist which have not yet been attracted by the objects.

15. A capturing method according to claim 11, wherein the first center is a center of gravity of the plural attraction ports that have not yet attracted by the objects.
